# FASCICULE DE BREVET EUROPEEN

(11) **EP 2 227 288 B1**
(45) Date de publication et mention de la délivrance du brevet: **30.03.2016**
(21) Numéro de dépôt: 08865773.9
(22) Date de dépôt: 11.12.2008
(51) Int. Cl.: A61M 39/10, A61B 1/00

(54) **APPAREILLAGE MEDICAL AVEC SYSTEME DE CONNEXION ET DE DECONNEXION POUR UN INSTRUMENT MEDICAL SEPARABLE**
MEDIZINISCHES GERÄT MIT EINEM ANSCHLUSS- UND TRENNSYSTEM FÜR EIN ABNEHMBARES MEDIZINISCHES INSTRUMENT
MEDICAL APPARATUS WITH A SYSTEM OF CONNECTION AND DISCONNECTION FOR A SEPARABLE MEDICAL INSTRUMENT

(30) Priorité: 11.12.2007 FR 0759713
(43) Date de publication de la demande: 15.09.2010
(73) Titulaire: Axess Vision Technology, 37000 Tours (FR)
(72) Inventeur: MATHIEU, Nicolas, F-69130 Ecully (FR)
(74) Mandataire: Thibault, Jean-Marc
(86) Numéro de dépôt international: PCT/FR2008/052277
(87) Numéro de publication internationale: WO 2009/080996

(56) Documents cités:
- WO-A-2006/116457
- FR-A- 2 794 535
- GB-A- 2 411 488
- US-A1- 2005 177 027

## Description

La présente invention concerne le domaine technique des appareillages médicaux au sens général, et elle vise plus précisément le domaine technique des appareillages comportant un instrument médical séparable par rapport à un support d'actionnement.

L'objet de l'invention concerne plus précisément un appareillage médical dont le support d'actionnement est destiné à être réutilisé alors que l'instrument médical qui est en contact avec les tissus ou organes humains relève d'un usage unique, multiple pour un même patient.

Dans l'état de la technique, il est connu de nombreux appareillages médicaux mettant en oeuvre un instrument médical à usage unique. Par exemple, dans le domaine de l'endoscopie, le brevet US 4 919 112 décrit un instrument tubulaire souple monté à l'aide d'un système de connexion et de déconnexion sur un support d'actionnement formant une poignée. L'instrument tubulaire souple comporte un raccord fileté incluant un levier articulé à chaque extrémité duquel est fixé un câble fixé à l'extrémité libre de l'instrument tubulaire souple pour permettre son pliage ou béquillage.

Cet instrument tubulaire souple est fixé sur la poignée à l'aide d'une bague filetée coopérant avec le raccord fileté. La poignée comporte une paire de tringles d'actionnement commandées en déplacement linéaire synchronisé alterné, de manière à appuyer successivement sur l'une ou l'autre des extrémités du levier d'actionnement des câbles. Ainsi, l'un des câbles subit un déplacement dans un sens, alors que l'autre câble subit un déplacement dans l'autre sens. Il doit être noté qu'il n'existe pas dans cette solution de liaison mécanique entre les câbles et les tringles d'actionnement, dans la mesure où ces dernières assurent uniquement un mouvement de poussée. Cette solution présente l'inconvénient de limiter les mouvements mécaniques transmis à l'instrument médical.

De même, la demande de brevet US 2005/177027 décrit un endoscope comportant des moyens de connexion électriques entre un tube d'insertion et une tête d'observation. La tête d'observation comporte une électrode annulaire reliée à des diodes électroluminescentes implantées à l'extrémité libre de la tête. Cette électrode annulaire est composée d'un empilage de trois feuilles de connexion et se trouve disposée à l'arrière de la tête, de manière à être en contact électrique avec des électrodes s'étendant à l'extrémité du tube d'insertion.

La tête d'observation comporte une bague d'assemblage pourvue intérieurement d'un double filetage. Le tube d'insertion est pourvu à son extrémité libre d'un filetage engagé axialement à l'intérieur de la bague qui est entrainée en rotation pour assurer l'assemblage par vissage du tube d'insertion sur la tête d'observation. En fin de course d'assemblage, les électrodes du tube d'insertion sont en contact électrique avec l'électrode de manière à assurer l'alimentation électrique des diodes luminescentes.

Ce document décrit un endoscope comportant un système pour assurer une connexion électrique par le rapprochement entre une tête d'observation et un tube d'insertion, à l'aide d'un système vis/écrou. Ce document ne décrit pas un système de connexion et de déconnexion permettant d'assurer une véritable liaison mécanique facilement et rapidement démontable entre un instrument médical et un support d'actionnement.

De même, le document GB 2411488 décrit un système d'endoscope comportant un système optique d'endoscope couplé de manière démontable à une partie caméra. Le système de couplage permet le passage à travers une fenêtre, à la fois du faisceau image et du faisceau lumineux.

Le document WO 2006/116457 décrit une sonde de cryochirurgie détachable comportant une sonde réutilisable pourvue d'une partie à usage unique munie d'un collier de rupture.

L'objet de l'invention vise à remédier aux inconvénients de l'état de la technique, en proposant un nouvel appareillage médical comportant un système de connexion et de déconnexion pour un instrument médical séparable, permettant d'assurer une véritable liaison mécanique démontable entre un instrument médical et un support d'actionnement.

Pour attendre un tel objectif, l'appareillage médical, selon l'invention, est conforme à la revendication 1.

Le système de connexion et de déconnexion est adapté pour séparer facilement l'instrument médical par rapport au support en procédant à un mouvement en sens contraire à celui ayant permis d'obtenir la connexion. Ainsi, dans la position connectée, le support et l'instrument médical sont déplacés relativement en rotation contraire pour entraîner en rotation le mécanisme d'engagement qui est adapté pour dégager la tête de la tige du crochet de manière qu'un mouvement d'écartement axial entre le support et l'instrument médical permet de déconnecter ce dernier du support d'actionnement.

Selon une variante de réalisation, au moins un crochet possède un logement de réception pour une tête de la tige pour assurer une liaison en translation entre la tige et le crochet d'actionnement.

Selon une autre variante de réalisation, au moins un crochet possède un logement de forme prismatique apte à recevoir une tête de tige prismatique complémentaire pour assurer une liaison en translation et/ou en rotation entre la tige et le crochet d'actionnement.

De préférence, le crochet possède une rainure de positionnement pour la tige, aménagée entre le logement de réception de la tête et l'extrémité libre du crochet.

Selon l'exemple de réalisation illustré, le mécanisme d'engagement est monté pour s'étendre, en position d'attente de connexion, à l'intérieur du crochet avec le dégagement situé à l'extérieur des crochets, de manière à recevoir la tête d'une tige à la suite du mouvement de rapprochement, le mécanisme d'engagement s'étendant, après rotation, à l'extérieur du crochet qui se trouve positionné à l'intérieur du dégagement afin de pouvoir coulisser librement.

Selon une variante avantageuse de réalisation, l'embout de raccordement est pourvu d'au moins un organe de raccord optique, électrique, fluidique ou pour passage mécanique, et en ce que le guide du connecteur est pourvu d'au moins un organe de raccord complémentaire respectivement optique, électrique, fluidique ou pour passage mécanique, adapté pour être aligné avec l'organe de raccord de l'embout, à la suite de la liaison mécanique entre la tige et le crochet d'actionnement.

Par exemple, l'organe de raccord traverse le mécanisme d'engagement à l'aide d'un dégagement adapté pour que ledit organe ne se trouve pas déplacé par le mécanisme d'engagement lors de sa rotation, ledit organe s'établissant sensiblement au niveau de l'extrémité libre du crochet.

Selon une autre caractéristique de l'objet de l'invention, le support comporte un corps découpé pour présenter une série de barrettes dont au moins une est mobile linéairement par rapport aux autres barrettes, afin de constituer une glissière faisant partie du système d'actionnement d'un crochet.

Avantageusement, chaque barrette formant glissière est pourvue d'un crochet à l'une de ses extrémités tandis que son autre extrémité est reliée à un organe d'actionnement en translation.

Selon un exemple de réalisation, au moins une barrette intègre un système d'actionnement en rotation et/ou en translation.

Selon un autre exemple de réalisation, au moins une barrette assure le positionnement d'un organe de raccord optique, électrique, fluidique ou pour un passage mécanique.

Avantageusement, le connecteur et l'embout de raccordement comportent des moyens d'indexation angulaire permettant leur alignement dans une position prédéterminée pour leur rapprochement axial.

Selon une caractéristique préférée de réalisation, le connecteur et l'embout comportent des moyens de guidage en coulissement pour le rapprochement axial, puis en rotation jusqu'à une position de butée correspondant à la position de connexion du système de connexion et de déconnexion.

De préférence, le connecteur et l'embout comportent des moyens de verrouillage du connecteur et de l'embout dans leur position de connexion.

Selon un exemple de réalisation, les moyens de verrouillage comportent un témoin de verrouillage à usage unique porté par le connecteur, et qui après la déconnexion de l'instrument à la suite de son utilisation, interdit une nouvelle connexion de l'instrument au support.

Selon un autre exemple de réalisation, les moyens de verrouillage comportent un témoin de verrouillage réutilisable porté par le connecteur ou par l'embout de raccordement, et qui après la déconnexion de l'instrument, permet une nouvelle connexion de instrument au support.

Par exemple, la tige d'actionnement est un élément souple tel qu'un câble ou un élément rigide tel qu'un axe rigide.

A titre d'exemple d'application, l'instrument médical séparable constitue tout ou partie d'un outil de chirurgie, un cathéter, un endoscope ou une sonde.

Selon une application avantageuse, l'instrument médical est un endoscope ou cathéter comportant une gaine tubulaire fixée sur le connecteur et à l'extrémité de laquelle est fixée au moins une tige d'actionnement pour assurer l'orientation de la tête de la gaine.

Avantageusement, la gaine tubulaire comporte au moins un organe de raccord optique, électrique, fluidique ou pour passage mécanique, à usage unique et complémentaire d'au moins un organe de raccord respectivement optique, électrique, fluidique ou pour passage mécanique, monté dans l'embout de raccordement.

Diverses autres caractéristiques ressortent de la description faite ci-dessous en référence aux dessins annexés qui montrent, à titre d'exemples non limitatifs, des formes de réalisation de l'objet de l'invention.
Les **Figures 1** et **2** sont des vues en perspective d'un appareillage médical conforme à l'invention illustré respectivement dans une position de déconnexion et une position de connexion.
La **Figure 3** est une vue en coupe élévation d'un appareillage médical en position de déconnexion.
La **Figure 4** est une vue en coupe transversale prise sensiblement selon les lignes IV-IV de la **Fig. 3****.**
Les **Figures 5** et **6** sont des vues en perspective partielle de l'appareillage médical en position avant connexion.
La **Figure 6A** est une vue montrant uniquement le corps du support.
La **Figure 7** est une vue en coupe élévation montrant un appareillage médical dans une position intermédiaire de connexion.
La **Figure 8** est une vue en perspective de l'appareillage médical dans la position intermédiaire illustrée à la **Fig. 7****.**
Les **Figures 9** et **10** sont des vues en coupe élévation décalée angulairement montrant l'appareillage médical en position de connexion.
La **Figure 11** est une vue en coupe transversale prise sensiblement selon les lignes XI-XI de la **Fig. 9****.**
Les **Figures 12** et **13** sont des vues en perspective de l'appareillage médical en position de connexion respectivement avant et après translation d'une tige d'actionnement.

Tel que cela ressort des Figures, l'objet de l'invention concerne un appareillage médical **1** comportant au moins un instrument médical séparable **2** et un support ou un bloc d'actionnement **3** se présentant de préférence sous la forme d'une poignée ou d'un bras robotisé. L'instrument médical **2** constitue par exemple tout ou partie d'un outil de chirurgie, un cathéter, un endoscope, une sonde.

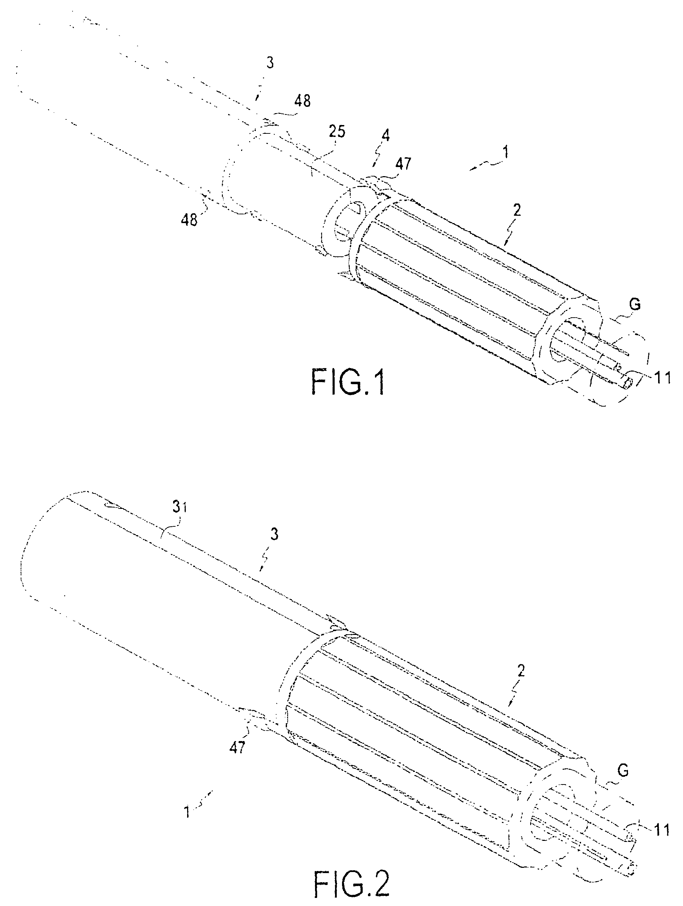

L'appareillage médical **1** comporte un système de connexion et de déconnexion **4** entre au moins un instrument médical **2** et le support **3,** adapté pour assurer rapidement une liaison au moins mécanique temporaire, tout en offrant l'avantage de permettre une séparation facile de l'instrument médical **2.** Ainsi, l'instrument médical **2** et le support **3** sont manoeuvrés relativement l'un par rapport à l'autre pour passer d'une position de déconnexion **(****Fig. 1****)** à une position de connexion **(****Fig. 2****).** Après l'utilisation de l'instrument médical **2,** le support **3** et l'instrument médical **2** sont manoeuvrés pour passer de la position de connexion à la position de déconnexion pour permettre le changement de l'instrument médical **2.** De préférence, l'instrument médical **2** est à usage unique, multiple pour un même patient.

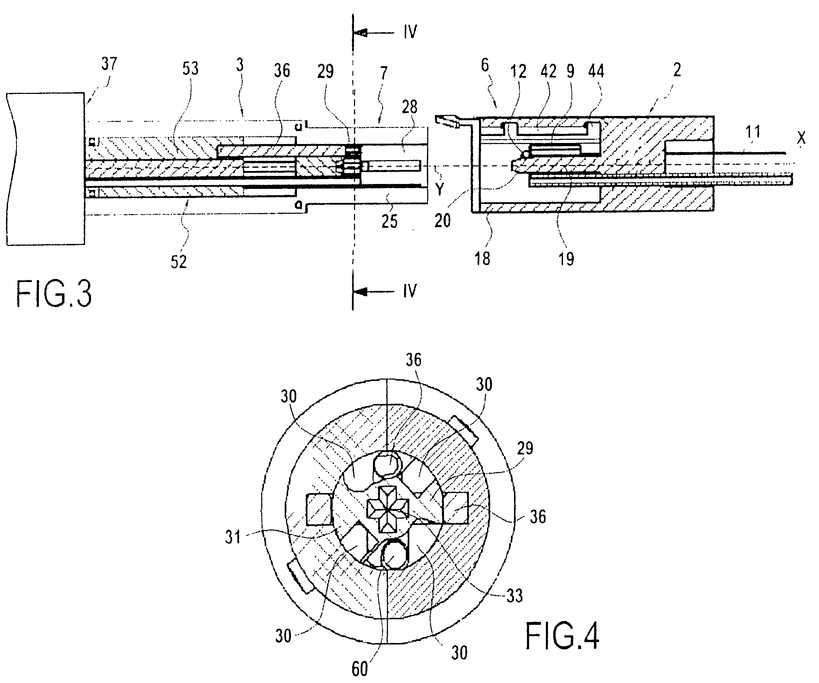

Tel que cela ressort plus précisément des **Fig. 3** à **6****,** le système de connexion et déconnexion **4** comporte un connecteur **6** d'axe longitudinal **X** porté par l'instrument médical **2** et destiné à coopérer avec un embout de raccordement **7** d'axe longitudinal **Y** porté par le support **3.** Le connecteur **6** comporte un guide **9** pour au moins une, et dans l'exemple illustré, trois tiges d'actionnement **11** se présentant sous une forme souple pour former un câble, ou rigide pour former un axe ou une tringle. Dans l'exemple de réalisation, l'instrument médical **2** comporte une tige rigide **11** et un jeu de deux tiges d'actionnement souple **11** dont les extrémités non représentées sont destinées à être reliées à des moyens adaptés au type d'instrument médical **2.** Par exemple, une tige **11** est connectée à un outil tandis que les deux autres tiges **11** sont reliées à une gaine souple **G** pour permettre son pliage, son béquillage, son extension, sa rétraction ou générer une pression ou dépression agissant directement ou indirectement.

Chaque tige d'actionnement **11** est retenue élastiquement dans le guide **9** par une tête **12** faisant saillie à l'extrémité libre du guide **9.** Chaque extrémité d'une tige **11** est pourvue d'une tête **12** de forme par exemple demi-hémisphérique ou prismatique s'étendant en saillie par rapport à la face transversale d'extrémité **9**₁ du guide **9.** Le guide **9** qui possède une forme cylindrique est aménagé pour présenter au moins une, et dans l'exemple illustré, trois rainures **14** de passage chacune pour une tige **11.** Chaque rainure **14** traverse de part en part le guide **9** selon l'axe longitudinal **X** et débouche à la périphérie du guide **9.** Avantageusement, chaque rainure **14** est aménagée pour s'étendre selon un segment circulaire centré sur l'axe longitudinal **X** en délimitant avec la périphérie du guide, une paroi **15** de retenue radiale pour la tige **11** engagée dans ladite rainure 14. Dans l'exemple de réalisation illustré, deux rainures de passage **14** s'étendent de manière symétriquement opposée par rapport à l'axe longitudinal **X,** de manière à pouvoir agir en deux points d'une section radiale par exemple de la gaine **G.** Bien entendu, il peut être envisagé une position angulaire différente des rainures **14** ainsi qu'un nombre différent en fonction du nombre de tiges d'actionnement **11** mises en oeuvre.

Un ressort de rappel **17** permet de solliciter les tiges **11** afin que les têtes **12** se trouvent maintenues en appui contre la face transversale d'extrémité **9₁**. Dans l'exemple illustré, le ressort **17** est interposé entre la face transversale **9₂** du guide opposée à la face transversale d'extrémité **9₁** et le connecteur **6** auquel les tiges **11** sont reliées mécaniquement. Il est à noter que le guide **9** se trouve maintenu en translation et en rotation par la pression du ressort **17** agissant sur les têtes **12** en saillie, ce qui permet de maintenir le connecteur dans une position d'attente de connexion.

Avantageusement, le connecteur **6** se présente sous la forme d'un corps aménagé pour présenter sur sa face avant, un manchon tubulaire d'emboîtement **18** à l'intérieur duquel est monté le guide **9** sur une colonnette **19** qui s'étend à partir du corps du connecteur, au centre du manchon et coaxialement au manchon **18.**

Selon une autre caractéristique, le connecteur **6** est pourvu d'un organe **20** d'accouplement en rotation. Dans l'exemple illustré, cet organe d'accouplement **20** est réalisé par une forme prismatique mâle aménagée à l'extrémité libre de la colonnette **19.** Ainsi cette forme prismatique mâle s'étend en saillie par rapport au guide **9** mais en retrait par rapport à l'extrémité libre du manchon d'accouplement **18.**

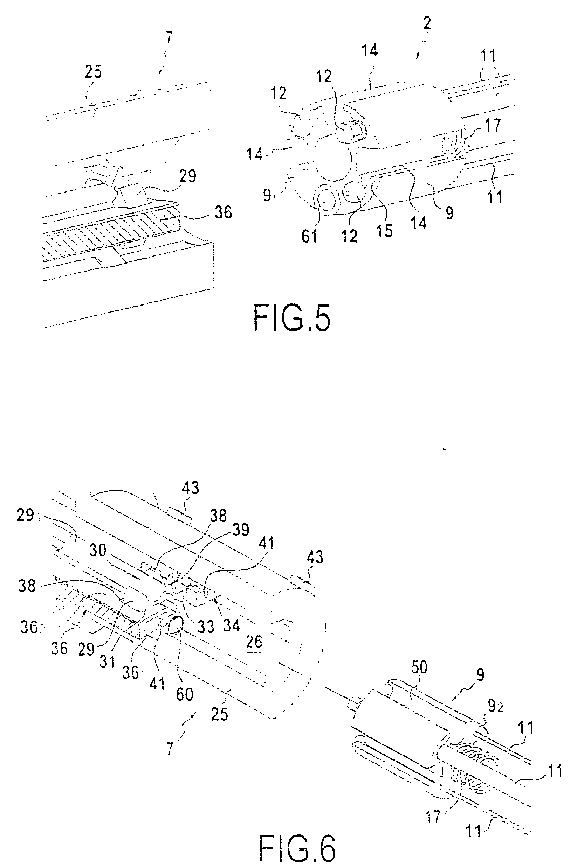

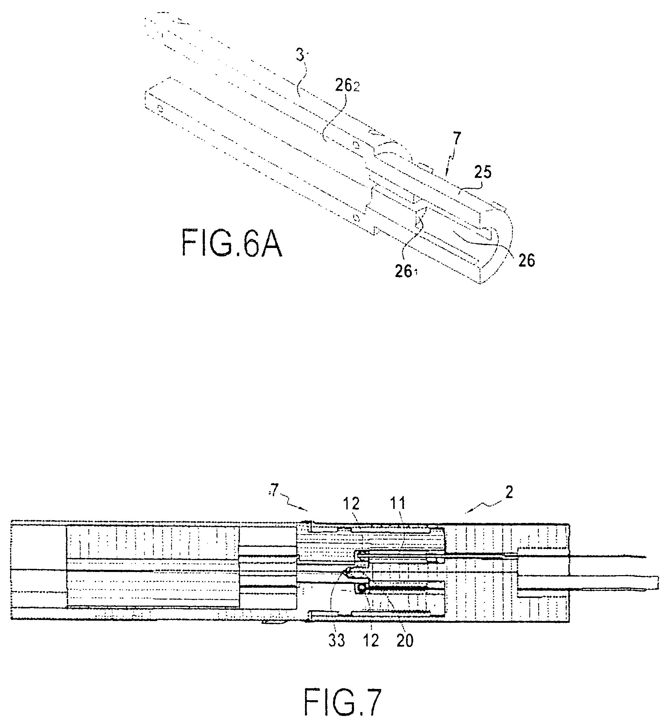

Le connecteur **6** est destiné à coopérer avec l'embout de raccordement **7** du support **3.** A cet effet, l'embout de raccordement **7** présente un nez d'accouplement tubulaire **25** destiné, comme cela sera expliqué dans la suite de la description, à venir s'engager entre le manchon tubulaire **18** et le guide **9** du connecteur **6.** Ce nez d'accouplement **25** délimite intérieurement un alésage **26** dans lequel est monté au moins un mécanisme d'engagement telle une came d'engagement **29** guidée en rotation autour de l'axe **Y.** La came **29** présente la forme d'un disque prolongé par un fût de montage **29₁.** La came **29** est montée en butée contre le fond **26₁** de l'alésage **26 (****Fig. 6A****)** qui débouche dans un autre alésage **26₂** coaxial et délimité par un corps tubulaire **3₁** du support **3.** La came **29** délimite au moins un, et dans l'exemple illustré, trois dégagements **30** pour la réception chacun d'une tête **12** d'une tige **11.** Les dégagements **30** s'ouvrent sur la face transversale d'extrémité **31** de la came **(****Fig. 4** et **6****).** Bien entendu, les dégagements **30** sont répartis selon une disposition angulaire identique de celle des têtes **12** des tiges qui sont destinées à pénétrer ensemble à l'intérieur des dégagements **30** par un simple mouvement de translation comme cela sera décrit dans la suite de la description.

La came **29** est équipée d'un organe d'accouplement en rotation **33** complémentaire de l'organe d'accouplement en rotation **20** présenté par le connecteur **6.** Selon l'exemple illustré, cet organe d'accouplement en rotation **33** présente une forme prismatique femelle aménagée sur la face transversale **31** de la came et complémentaire de la forme prismatique mâle **20.**

L'embout de raccordement **7** comporte également au moins un, et dans l'exemple illustré, trois crochets **36** reliés à un système d'actionnement **37.** Les trois crochets **36** sont montés pour s'étendre à l'intérieur de l'alésage **26.** Chaque crochet **36** se présente sous la forme d'un corps allongé, s'étendant parallèlement à l'axe **Y.** Dans l'exemple illustré, deux crochets **36** sont disposés de manière symétriquement opposée par rapport à l'axe **Y.** Chaque crochet **36** comporte un logement **38** de réception pour une tête **12** d'une tige **11.** Chaque logement **38** s'ouvre radialement à l'intérieur de l'alésage **26** de manière à être bordé d'un côté par une branche radiale distale **36₁** s'étendant parallèlement et à distance d'une branche radiale proximale **36₂**.

Chaque logement **38** possède une forme adaptée à la tête **12** qu'il reçoit. Ainsi, l'un des crochets **36** possède un logement **38** de forme prismatique complémentaire de la forme prismatique de la tête **12.** Les deux autres crochets **36** présentent dans l'exemple illustré, un logement **38** de forme rectangulaire adaptée pour recevoir la tête hémisphérique qui est destinée à venir en appui par sa base sur la branche radiale proximale **36₂**.

Dans une position neutre ou d'attente de connexion illustrée aux Figures **4** à **6****,** les crochets **36** sont disposés de manière que les logements **38** se trouvent alignés dans un même plan transversal à l'axe **Y.** Avantageusement, la came **29** est montée de manière que son chemin circulaire de rotation qui s'établit dans un plan perpendiculaire à l'axe **Y,** débouche dans les logements **38.** Il doit être compris que dans cette position neutre, la came **29** est engagée dans au moins certains des logements **38** tandis que les dégagements **30** de la came s'étendent en dehors des crochets **36** pour permettre la réception des têtes **12** qui sont destinées à être déplacées par la came **29.** En effet, la came **29** est aménagée pour recevoir les têtes **12** et les déplacer angulairement selon les deux sens de rotation de la came **29** comme cela sera expliqué dans la suite de la description.

Il est à noter qu'il peut être prévu des moyens pour maintenir en position la came **29** dans cette position d'attente de connexion. Par exemple, ces moyens de maintien peuvent être réalisés par une vis à bille pointeau. Avantageusement, la came **29** empêche le coulissement des crochets **36** et permet de positionner les crochets dans leur position d'attente de connexion.

La mise en oeuvre du système de connexion et de déconnexion **4** décrit ci-dessus découle directement de la description qui précède.

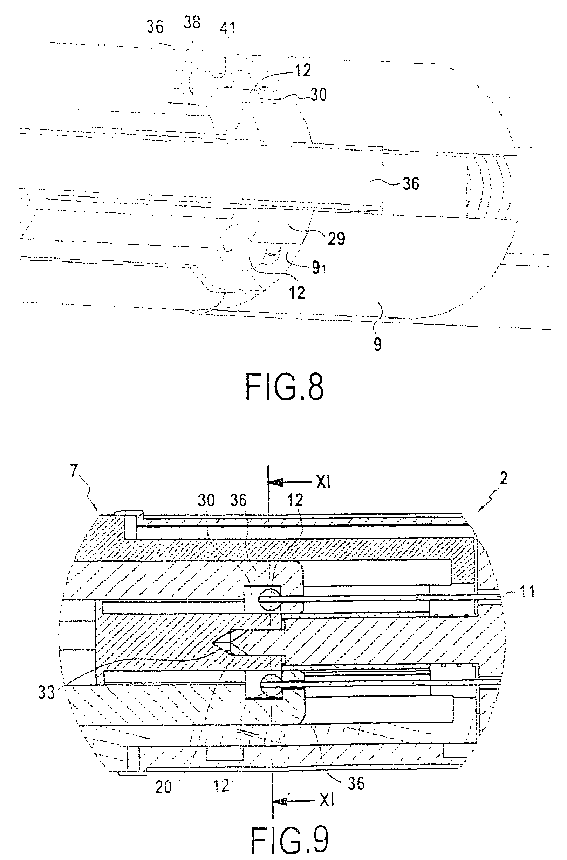

L'assemblage d'un instrument médical **2** à un support d'actionnement **3** consiste à partir d'une position d'attente de connexion, à assurer l'alignement axial et angulaire entre le connecteur **6** et l'embout de raccordement **7,** avant de les rapprocher axialement l'un par rapport à l'autre. Le mouvement de rapprochement axial conduit à l'engagement du nez d'accouplement **25** entre le manchon **18** et le guide **9** de l'instrument médical **2.** Le support **3** et l'instrument médical **2** sont positionnés angulairement entre eux de manière que chaque tête **12** se trouve située dans l'alignement d'un dégagement **30** de la came **29.** Le mouvement axial est réalisé jusqu'à obtenir l'appui entre le guide **9** et la came **29.** La poursuite du déplacement axial du connecteur **6** conduit à la compression du ressort **17** et au décollement des têtes **12** par rapport à la face transversale **9₁** du guide **9,** tel que cela ressort plus précisément des **Fig. 7** et **8****.** Dans cette position, la forme prismatique mâle **20** du connecteur **6** est engagée dans la forme prismatique femelle **33** de la came. Dans cette position, chaque tête **12** s'étend en saillie par rapport à la face transversale **9₁** et se trouve positionnée dans un dégagement **30** de la came, en regard d'un logement **38** d'un crochet. En d'autres termes, les têtes **12** sont placées dans le plan de rotation de la came **29.**

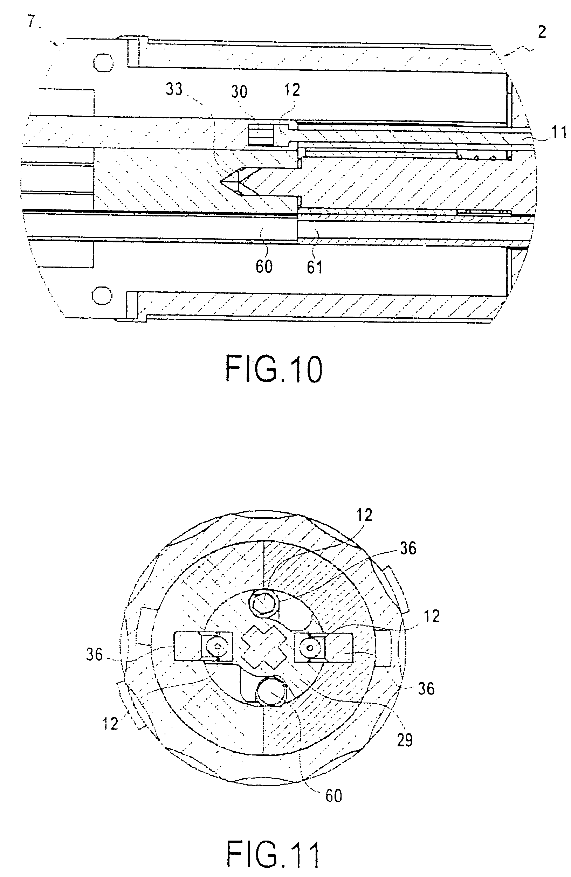

Un mouvement de rotation relatif entre le connecteur **6** et le support **3** conduit à la rotation de la came **29** qui entraîne en rotation chaque tête **12** jusqu'à venir coopérer avec le crochet voisin **36.** Au terme de ce mouvement de rotation, chaque tête **12** est positionnée à l'intérieur d'un logement **38** du crochet **(****Fig. 9** à **12****).** Dans cette position, la came **29** s'étend à l'extérieur du crochet **36** de sorte que le crochet **36** se trouve positionné à l'intérieur du dégagement **30** de la came, permettant ainsi au crochet **36** de pouvoir coulisser librement. Le système de connexion et de déconnexion **4** permet ainsi d'assurer une liaison mécanique entre l'instrument médical **2** et le support d'actionnement **3,** par l'engagement de chaque tête **12** d'une tige dans un crochet **36.**

Il ressort de la description qui précède, que le système de connexion et de déconnexion **4** permet d'obtenir l'assemblage mécanique de l'instrument médical **2** avec le support **3,** en assurant, après leur alignement angulaire et axial, leur rapprochement axial suivi d'une rotation relative. Bien entendu, le passage de la position connectée à la position déconnectée consiste à procéder de manière inverse, c'est-à-dire à effectuer une rotation relative entre le support **3** et l'instrument médical **2,** dans un sens contraire afin que la came **29** entraîne en rotation les têtes **12** pour les faire sortir des logements **38** des crochets **36.** Au terme de cette rotation, les têtes **12** sont situées en dehors des crochets **36** en s'étendant à l'intérieur des dégagements **30.** Un mouvement d'écartement relatif axial entre l'instrument médical **2** et le support **3** permet leur séparation.

Il doit être noté que la came **29** assure l'engagement des têtes **12** dans les crochets **36** selon un sens de rotation, et leur désengagement lors d'un mouvement de rotation de sens contraire. Ainsi, la came **29** comporte des surfaces de butée délimitant de part et d'autre les dégagements **30** et adaptées pour assurer le déplacement en rotation des têtes selon les deux sens de rotation de la came. Selon une variante de réalisation, la came **29** est pourvue d'un éjecteur **39** pour assurer la sortie de la tête **12** de forme prismatique par rapport à son logement **38.**

En fonction de la forme de la tête **12,** il peut être obtenu une liaison en translation et/ou en rotation entre la tige **11** et le crochet **36** associé. De préférence, chaque crochet **36** possède une rainure **41** de positionnement pour la tige **11,** aménagée entre le logement de réception **38** et l'extrémité libre du crochet. Cette rainure **41** permet de maintenir en position droite la tige **11** sans la courber, avec la tête **12** centrée dans son logement **38.**

Selon une variante de réalisation, le connecteur **6** et l'embout de raccordement **7** comportent des moyens d'indexation angulaire permettant leur alignement dans une position prédéterminée pour leur rapprochement axial. Ces moyens d'indexation non représentés permettent à l'utilisateur d'orienter convenablement angulairement l'instrument médical **2** par rapport au support **3,** afin d'assurer l'alignement entre les têtes **12** des tiges et les dégagements **30** de la came **29.** Par exemple, ces moyens d'indexation peuvent être réalisés par des marquages réalisés sur le connecteur **6** et l'embout de raccordement **7** et dont leur alignement correspond à la position de rapprochement axial entre l'instrument médical **2** et le support **3.**

Selon une variante préférée de réalisation, le connecteur **6** et l'embout **7** comportent des moyens de guidage en coulissement pour le rapprochement axial puis en rotation jusqu'à une position de butée correspondant à la position de connexion du système de connexion et de déconnexion **4.** Dans l'exemple de réalisation illustré, les moyens de guidage comportent une rainure **42** aménagée sur la face interne du manchon **18** et parallèlement à l'axe longitudinal **X** du connecteur. De façon complémentaire, le nez d'accouplement tubulaire **25** est muni sur sa face externe de deux ergots **43** alignés selon une droite parallèle à l'axe **Y** du support **3.** Lors de la connexion de l'instrument médical **2,** le support **3** est positionné angulairement par rapport à l'instrument médical de manière à obtenir l'engagement des deux ergots dans la rainure **18.** Bien entendu, ce positionnement correspond à la position de rapprochement axial permettant l'engagement des têtes **12** dans les dégagements **30.** Il est à noter que les moyens d'indexation angulaire sont réalisés pour visualiser ce positionnement angulaire entre le support **3** et l'instrument médical **2.**

Les moyens de guidage en rotation sont réalisés par deux rainures **44** réalisées sur la face interne du manchon **18** selon des secteurs angulaires situés dans des plans parallèles entre eux et perpendiculaires à l'axe longitudinal **X.** Ces rainures **44** débouchent dans la rainure **42** à l'endroit où sont positionnés les ergots **43** à la fin du mouvement de rapprochement axial entre l'instrument médical **2** et le support **3.** La longueur des rainures **44** est déterminée pour obtenir la mise en butée des ergots **43** sur le fond des rainures, correspondant à la position de connexion du système de connexion et de déconnexion **4.**

Selon une variante de réalisation, le connecteur **6** et l'embout **7** comportent des moyens de verrouillage du connecteur et de l'embout dans leur position de connexion. En d'autres termes, le connecteur **6** et l'embout **7** comportent des moyens permettant au système **4** de passer de sa position de connexion à sa position de déconnexion. Par exemple, ces moyens de verrouillage peuvent être réalisés par des lumières aménagées dans le fond des rainures **44** pour permettre de constituer une butée anti-retour des ergots **43** dans un sens contraire permettant d'amener les ergots dans leur position de connexion. Bien entendu, il peut être prévu de réaliser différemment les moyens de verrouillage par la mise en oeuvre par exemple comme illustré aux **Fig. 1** et **2****,** de pattes **47** faisant saillie du manchon **18** et destinées à coopérer en position de connexion, avec des cavités **48** aménagées à l'extérieur du corps **3₁** du support **3.**

Il est à noter qu'il peut être prévu que l'instrument médical **2** possède un témoin d'utilisation permettant de savoir si l'instrument médical a été ou non connecté à un support **3.**

Selon une variante de réalisation, les moyens de verrouillage comportent un témoin de verrouillage permettant de montrer à l'utilisateur que l'instrument médical **2** est en position connectée. Selon une variante de réalisation, ce témoin de verrouillage est à usage unique. Par exemple, ce témoin de verrouillage est porté par l'instrument médical et se trouve détérioré après la déconnexion de l'instrument médical **2** par rapport au support **3.** Dans ces conditions, l'instrument médical **2** ne peut plus être monté dans une position verrouillée sur le support **3** dans la mesure où le témoin de verrouillage a été détérioré. Par exemple, ce témoin de verrouillage à usage unique peut être réalisé par les pattes **47** qui sont adaptées pour se casser lors de la déconnexion de l'instrument médical **2** par rapport au support **3.** Selon une autre variante de réalisation, le témoin de verrouillage porté par le connecteur ou par l'embout de raccordement peut être à caractère réutilisable pour autoriser, après la déconnexion de l'instrument médical **2,** une nouvelle connexion de l'instrument médical **2** au support **3.** Cette solution offre l'avantage de pouvoir utiliser, à plusieurs reprises, pour un même patient, un instrument médical avec un support **3** qui est également utilisé avec un autre instrument médical.

Tel que cela ressort de la description qui précède, le système de connexion et de déconnexion **4** permet d'assurer une liaison mécanique entre le support **3** et l'instrument médical **2** permettant de transmettre un mouvement de translation dans les deux sens et/ou un mouvement de rotation. Il est à noter que dans le cas d'une liaison en translation, le guide **9** est pourvu de rainures **50** parallèles à l'axe longitudinal **X.** Ces rainures **50** sont adaptées pour permettre de recevoir voire guider un crochet **36** lors de ses mouvements de translation.

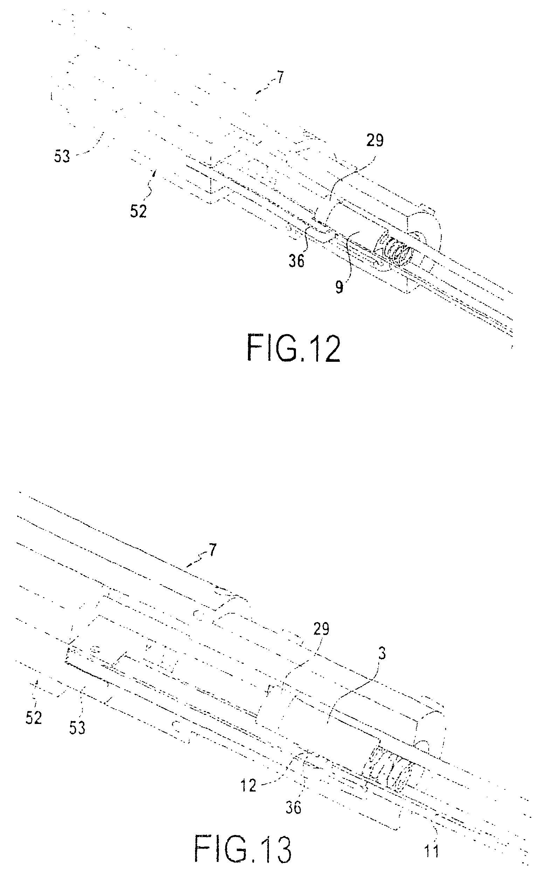

Selon une variante de réalisation, le système d'actionnement **37** des crochets comporte un corps découpé **52** monté à l'intérieur du corps **3₁** de l'embout **7.** Ce corps **52** est découpé pour présenter une série de barrettes **53** dont au moins une est mobile linéairement par rapport aux autres barrettes, afin de constituer une glissière d'actionnement d'un crochet **36.** Dans l'exemple illustré, chaque barrette formant glissière est pourvue à l'une de ses extrémités, d'un crochet **36 (****Fig. 13****)** tandis que son autre extrémité est reliée à un organe d'actionnement en translation non représenté mais de tout type connu en soi. Ainsi, à chaque barrette **53** peut être reliée par exemple directement ou par l'intermédiaire d'un système de transformation de mouvement, à un moteur ou à un piston. Par exemple, le système de transformation de mouvement peut être par exemple du type vis sans fin/écrou ou pignon crémaillère, avec l'un des éléments montés solidaire de la barrette **53.** Selon un autre exemple de réalisation, il peut être prévu qu'une barrette **53** intègre un système d'actionnement en rotation et/ou en translation. Ainsi, il peut être prévu que des barrettes **53** soient mobiles relativement les unes au autres en se guidant en translation les unes par rapport aux autres. Selon cette variante de réalisation, les barrettes **53** possèdent une forme délimitée par des faces planes. Il peut être également prévu que certaines de ces barrettes **53** soient fixes.

Dans la description qui précède, le système de connexion et de déconnexion **4** permet d'assurer une liaison mécanique entre l'instrument médical **2** et le support d'actionnement **3.** Selon une variante de réalisation, il est à noter que le système de connexion et de déconnexion **4** permet d'assurer également un raccord de nature différente par exemple optique, électrique, fluidique ou assurer le raccordement pour un passage mécanique pour tout mécanisme.

Selon cette variante de réalisation, l'embout de raccordement **7** est pourvu d'au moins un organe **60** de raccord optique, électrique, fluidique ou pour un passage mécanique. Le guide **9** du connecteur **6** est également pourvu au moins d'un organe **61** de raccord complémentaire respectivement optique, électrique, fluidique ou pour un passage mécanique adapté pour être aligné avec l'organe de raccord **60** de l'embout à la suite de la liaison mécanique entre la tige **11** et le crochet d'actionnement **36.** Il est à noter que l'organe de raccord **60** traverse la came d'engagement **29** à l'aide d'un dégagement **30** adapté pour que ledit organe **60** ne se trouve pas déplacé par la came **29** lors de sa rotation **(****Fig. 4****).** De préférence, cet organe de raccord **60** s'établit sensiblement au niveau de l'extrémité libre du crochet **36** pour permettre d'obtenir un raccord ou une liaison avec l'organe de raccord complémentaire porté par l'instrument médical **2.** Il est à noter que cet organe de raccordement **60** est monté dans une barrette **53** montée fixe ou mobile, par exemple en translation, pour parfaire la liaison avec l'organe de raccord **61** complémentaire. Bien entendu, les organes de raccord **60, 61** peuvent être de tout type et de toute nature. Ces organes de raccords **60, 61** sont par exemple, des fibres optiques (associées ou non à des éléments optiques), des câbles électriques, des tubes de passage pour un fluide ou pour un organe, etc..

Il ressort de la description qui précède que le système de connexion et de déconnexion **4** peut assurer une ou plusieurs liaisons mécaniques en translation et/ou en rotation ainsi qu'une ou plusieurs liaisons optiques, électriques, fluidiques et/ou un ou plusieurs passages mécaniques. Bien entendu, le support **3** peut comporter une multitude de crochets **36** et d'organes de raccord **60** de manière à être adapté pour recevoir successivement un ou avantageusement, une série d'instruments médicaux **2** différents utilisant tout ou partie des liaisons mécaniques ou autres.

Dans les exemples décrits ci-dessus, le système de connexion et de déconnexion **4** comporte une seule came **29** pour permettre le raccordement de une ou plusieurs tiges d'actionnement. Bien entendu, il peut être prévu un support équipé de plusieurs cames d'engagement pour une ou plusieurs tiges d'actionnement.

L'invention n'est pas limitée aux exemples décrits et représentés car diverses modifications peuvent y être apportées sans sortir de son cadre.

## Revendications

1. Appareillage médical comportant un instrument médical **(2)** séparable par rapport à un support d'actionnement **(3),** le dispositif comportant un système de connexion et de déconnexion **(4)** entre l'instrument **(2)** et le support **(3),** comportant :
- un connecteur **(6)** porté par l'instrument et comportant un guide **(9)** pour au moins une tige d'actionnement **(11)** pourvue d'une tête **(12)** faisant saillie à l'extrémité libre du guide, le connecteur **(6)** étant pourvu d'un organe d'accouplement en rotation **(20),**
- et un embout de raccordement **(7)** porté par le support et comportant au moins un mécanisme d'engagement **(29),** ce mécanisme d'engagement **(29)** étant équipé d'un organe d'accouplement en rotation **(33)** complémentaire de l'organe d'accouplement en rotation **(20)** présenté par le connecteur **(6),** l'embout **(7)** et le connecteur **(6)** occupant une position de connexion à la suite de leur rapprochement axial conduisant à l'accouplement en rotation entre le connecteur **(6)** et le mécanisme d'engagement **(29), caractérisé en ce que** :
- la tête **(12)** de la tige d'actionnement **(11)** est de forme demi-hémisphérique ou prismatique, retenue élastiquement en appui sur une face transversale d'extrémité **(9₁)** du guide, par l'intermédiaire d'un ressort de rappel **(17),**
- l'embout **(7)** présente au moins un crochet **(36)** relié à un système d'actionnement **(37)** et possédant un logement **(38)** de réception pour la tête **(12)** de la tige pour assurer une liaison en translation entre la tige **(11)** et le crochet d'actionnement **(36),**
- le mécanisme d'engagement **(29)** est une came guidée en rotation et délimitant un dégagement **(30)** de réception pour la tête **(12)** de la tige d'actionnement **(11),** la came étant montée pour s'étendre en position d'attente de connexion, à l'intérieur du crochet **(36)** avec le dégagement **(30)** situé à l'extérieur des crochets de manière, à la suite du mouvement de rapprochement axial, à recevoir la tête **(12)** d'une tige **(11)** qui se trouve dégagée par rapport au guide **(9),** le rapprochement axial étant suivi par un mouvement de rotation entre le connecteur **(6)** et l'embout de raccordement **(7)** pour entraîner la rotation du mécanisme d'engagement **(29)** qui déplace la tête **(12)** de la tige jusqu'à sa coopération avec le crochet **(36)** en vue d'assurer une liaison mécanique de la tête **(12)** de la tige avec le crochet d'actionnement **(36),** le mécanisme d'engagement **(29)** s'étendant, après rotation, à l'extérieur du crochet **(36)** qui se trouve positionné à l'intérieur du dégagement **(30)** afin de pouvoir coulisser librement.

2. Appareillage médical selon la revendication 1, **caractérisé en ce qu'**au moins un crochet **(36)** possède un logement de forme prismatique apte à recevoir une tête **(12)** de tige prismatique complémentaire pour assurer une liaison en translation et/ou en rotation entre la tige **(11)** et le crochet d'actionnement **(36).**

3. Appareillage médical selon la revendication 2, **caractérisé en ce que** le crochet **(36)** possède une rainure **(41)** de positionnement pour la tige, aménagée entre le logement de réception **(38)** de la tête et l'extrémité libre du crochet.

4. Appareillage médical selon l'une des revendications 1 à 3, **caractérisé en ce que** l'embout de raccordement **(7)** est pourvu d'au moins un organe **(60)** de raccord optique, électrique, fluidique ou pour passage mécanique, et **en ce que** le guide **(9)** du connecteur est pourvu d'au moins un organe **(61)** de raccord complémentaire respectivement optique, électrique, fluidique ou pour passage mécanique, adapté pour être aligné avec l'organe **(60)** de raccord de l'embout, à la suite de la liaison mécanique entre la tige **(11)** et le crochet d'actionnement **(36).**

5. Appareillage médical selon la revendication 4, **caractérisé en ce que** l'organe de raccord **(60)** traverse le mécanisme d'engagement **(29)** à l'aide d'un dégagement **(30)** adapté pour que ledit organe **(60)** ne se trouve pas déplacé par le mécanisme d'engagement lors de sa rotation, ledit organe **(60)** s'établissant sensiblement au niveau de l'extrémité libre du crochet.

6. Appareillage médical selon l'une des revendications 1 à 5, **caractérisé en ce que** le support **(3)** comporte un corps découpé **(52)** pour présenter une série de barrettes **(53)** dont au moins une est mobile linéairement par rapport aux autres barrettes, afin de constituer une glissière faisant partie du système d'actionnement **(37)** d'un crochet **(36).**

7. Appareillage médical selon la revendication 6, **caractérisé en ce que** chaque barrette **(53)** formant glissière est pourvue d'un crochet **(36)** à l'une de ses extrémités tandis que son autre extrémité est reliée à un organe d'actionnement en translation.

8. Appareillage médical selon la revendication 6, **caractérisé en ce qu'**au moins une barrette **(53)** intègre un système d'actionnement en rotation et/ou en translation.

9. Appareillage médical selon la revendication 6, **caractérisé en ce qu'**au moins une barrette **(53)** assure le positionnement d'un organe de raccord optique, électrique, fluidique ou pour un passage mécanique.

10. Appareillage médical selon l'une des revendications 1 à 9, **caractérisé en ce que** le connecteur **(6)** et l'embout de raccordement **(7)** comportent des moyens d'indexation angulaire permettant leur alignement dans une position prédéterminée pour leur rapprochement axial.

11. Appareillage médical selon la revendication 10, **caractérisé en ce que** le connecteur **(6)** et l'embout **(7)** comportent des moyens **(42, 43, 44)** de guidage en coulissement pour le rapprochement axial, puis en rotation jusqu'à une position de butée correspondant à la position de connexion du système de connexion et de déconnexion **(4).**

12. Appareillage médical selon l'une des revendications 1 à 11, **caractérisé en ce que** le connecteur **(6)** et l'embout **(7)** comportent des moyens de verrouillage du connecteur et de l'embout dans leur position de connexion.

13. Appareillage médical selon la revendication 11, **caractérisé en ce que** les moyens de verrouillage **(42, 43, 44)** comportent un témoin de verrouillage à usage unique porté par le connecteur, et qui après la déconnexion de l'instrument à la suite de son utilisation, interdit une nouvelle connexion de l'instrument au support.

14. Appareillage médical selon la revendication 12, **caractérisé en ce que** les moyens de verrouillage comportent un témoin de verrouillage réutilisable porté par le connecteur **(6)** ou par l'embout de raccordement **(7),** et qui après la déconnexion de l'instrument **(2),** permet une nouvelle connexion de l'instrument au support **(3).**

15. Appareillage médical selon l'une des revendications 1 à 14, **caractérisé en ce que** la tige d'actionnement **(11)** est un élément souple tel qu'un câble ou un élément rigide tel qu'un axe rigide.

16. Appareillage médical selon l'une des revendications 1 à 15, **caractérisé en ce que** l'instrument médical séparable constitue tout ou partie d'un outil de chirurgie, un cathéter, un endoscope ou une sonde.

17. Appareillage médical selon la revendication 15, **caractérisé en ce que** l'instrument médical **(2)** est un endoscope ou cathéter comportant une gaine tubulaire **(G)** fixée sur le connecteur **(6)** et à l'extrémité de laquelle est fixée au moins une tige d'actionnement **(11)** pour assurer l'orientation de la tête de la gaine.

18. Appareillage médical selon la revendication 17, **caractérisé en ce que** la gaine tubulaire **(G)** comporte au moins un organe **(61)** de raccord optique, électrique, fluidique ou pour passage mécanique, à usage unique et complémentaire d'au moins un organe **(60)** de raccord respectivement optique, électrique, fluidique ou pour passage mécanique, monté dans l'embout de raccordement **(7).**

## Patentansprüche

1. Medizinisches Gerät, das ein medizinisches Instrument (2) aufweist, das gegenüber einem Betätigungsträger (3) abnehmbar ist, wobei das Gerät ein Anschluss- und Trennsystem (4) zwischen dem Instrument (2) und dem Träger (3) aufweist, umfassend:
- einen Verbinder (6), der von dem Instrument getragen ist und eine Führung (9) für mindestens eine Betätigungsstange (11) aufweist, die mit einem Kopf (12) versehen ist, der an dem freien Ende der Führung vorsteht, wobei der Verbinder (6) mit einem rotierenden Kupplungsorgan (20) versehen ist,
- und ein Anschlussstück (7), das von dem Träger getragen ist und mindestens einen Eingreifmechanismus (29) aufweist, wobei dieser Eingreifmechanismus (29) mit einem rotierenden Kupplungsorgan (33) ausgestattet ist, das komplementär zu dem rotierenden Kupplungsorgan (20) ist, das der Verbinder (6) aufweist, wobei das Anschlussstück (7) und der Verbinder (6) infolge ihrer axialen Annäherung eine Verbindungsposition einnehmen, die zu der rotierenden Kuppelung zwischen dem Verbinder (6) und dem Eingreifmechanismus (29) führt, **dadurch gekennzeichnet, dass**:
- der Kopf (12) der Betätigungsstange (11) von halb hemisphärischer oder prismatischer Form ist, der durch eine Rückstellfeder (17) elastisch in Anlage auf einer Querstirnfläche (9) der Führung gehalten wird,
- das Anschlussstück (7) mindestens einen Haken (36) aufweist, der mit einem Betätigungssystem (37) verbunden ist und eine Aussparung (38) zur Aufnahme für den Kopf (12) der Stange aufweist, um eine Verbindung in Translation zwischen der Stange (11) und dem Betätigungshaken (36) zu gewährleisten,
- der Eingreifmechanismus (29) eine Nocke ist, die drehbar geführt ist und einen Freiraum (30) zur Aufnahme für den Kopf (12) der Betätigungsstange (11) begrenzt, wobei die Nocke montiert ist, um sich in Verbindungswarteposition im Inneren des Hakens (36) zu erstrecken, mit dem Freiraum (30), der außerhalb der Haken derart angeordnet ist, um nach der axialen Annäherungsbewegung den Kopf (12) einer Stange (11) aufzunehmen, die gegenüber der Führung (9) ausgerückt ist, wobei auf die axiale Annäherung eine Drehbewegung zwischen dem Verbinder (6) und dem Anschlussstück (7) folgt, um die Drehung des Eingreifmechanismus (29) anzutreiben, der den Kopf (12) der Stange bis zu seinem Zusammenwirken mit dem Haken (36) verschiebt, um eine mechanische Verbindung des Kopfes (12) der Stange mit dem Betätigungshaken (36) zu gewährleisten, wobei sich der Eingreifmechanismus (29) nach der Drehung außerhalb des Hakens (36) erstreckt, der sich im Inneren des Freiraums (30) angeordnet befindet, um frei gleiten zu können.

2. Medizinisches Gerät nach Anspruch 1, **dadurch gekennzeichnet, dass** mindestens ein Haken (36) eine Aussparung von prismatischer Form aufweist, die geeignet ist, einen komplementären prismatischen Kopf (12) einer Stange aufzunehmen, um eine Verbindung in Translation und/oder in Drehung zwischen der Stange (11) und dem Betätigungshaken (36) zu gewährleisten.

3. Medizinisches Gerät nach Anspruch 2, **dadurch gekennzeichnet, dass** der Haken (36) eine Positionierungsnut (41) für die Stange aufweist, die zwischen der Aussparung zur Aufnahme (38) des Kopfes (12) und dem freien Ende des Hakens angeordnet ist.

4. Medizinisches Gerät nach einem der Ansprüche 1 bis 3, **dadurch gekennzeichnet, dass** das Anschlussstück (7) mit mindestens einem Organ (60) zum optischen, elektrischen Anschluss, zum Fluidanschluss oder zum Anschluss für einen mechanischen Durchgang versehen ist und dass die Führung (9) des Verbinders (61) mit mindestens einem komplementären Organ (60) zum optischen, elektrischen Anschluss, zum Fluidanschluss oder zum Anschluss für einen mechanischen Durchgang versehen ist, das geeignet ist, mit dem Organ (60) zum Anschluss des Anschlussstücks infolge der mechanischen Verbindung zwischen der Stange (11) und dem Betätigungshaken (36) ausgerichtet zu werden.

5. Medizinisches Gerät nach Anspruch 4, **dadurch gekennzeichnet, dass** das Organ zum Anschluss (60) den Eingreifmechanismus (29) mittels eines Freiraums (30) durchquert, der geeignet ist, damit das Organ (60) von dem Eingreifmechanismus bei seiner Drehung nicht verschoben wird, wobei sich das Organ (60) im Wesentlichen an dem freien Ende des Hakens anordnet.

6. Medizinisches Gerät nach einem der Ansprüche 1 bis 5, **dadurch gekennzeichnet, dass** der Träger (3) einen ausgeschnittenen Körper (52) aufweist, um eine Reihe von Stegen (53) aufzuweisen, wovon mindestens einer gegenüber den anderen Stegen linear beweglich ist, um eine Schiene zu bilden, die Teil des Betätigungssystems (37) eines Hakens (36) ist.

7. Medizinisches Gerät nach Anspruch 6, **dadurch gekennzeichnet, dass** jeder Steg (53), der eine Schiene bildet, mit einem Haken (36) an einem seiner Enden versehen ist, während sein anderes Ende mit einem Organ zum Betätigen in Translation verbunden ist.

8. Medizinisches Gerät nach Anspruch 6, **dadurch gekennzeichnet, dass** mindestens ein Steg (53) ein System zum Betätigen in Drehung und/oder in Translation integriert.

9. Medizinisches Gerät nach Anspruch 6, **dadurch gekennzeichnet, dass** mindestens ein Steg (53) das Positionieren eines Organs zum optischen, elektrischen Anschluss, zum Fluidanschluss oder zum Anschluss für einen mechanischen Durchgang gewährleistet.

10. Medizinisches Gerät nach einem der Ansprüche 1 bis 9, **dadurch gekennzeichnet, dass** der Verbinder (6) und das Anschlussstück (7) Mittel zur Winkelindexierung aufweisen, die ihr Ausrichten in eine vorbestimmte Position für ihre axiale Annäherung ermöglichen.

11. Medizinisches Gerät nach Anspruch 10, **dadurch gekennzeichnet, dass** der Verbinder (6) und das Anschlussstück (7) Mittel (42, 43, 44) zum Führen in Verschiebung für die axiale Annäherung und dann in Drehung bis zu einer Anschlagsposition aufweisen, die der Verbindungsposition des Anschluss- und Trennsystems (4) entspricht.

12. Medizinisches Gerät nach einem der Ansprüche 1 bis 11, **dadurch gekennzeichnet, dass** der Verbinder (6) und das Anschlussstück (7) Mittel zum Verriegeln des Verbinders und des Anschlussstücks in ihrer Verbindungsposition aufweisen.

13. Medizinisches Gerät nach Anspruch 11, **dadurch gekennzeichnet, dass** die Mittel zum Verriegeln (42, 43, 44) eine Verriegelungsanzeige zur Einweg-Verwendung aufweisen, die von dem Verbinder getragen ist und die nach dem Trennen des Instrumentes nach seiner Verwendung einen neuen Anschluss des Instrumentes an den Träger verbietet.

14. Medizinisches Gerät nach Anspruch 12, **dadurch gekennzeichnet, dass** die Mittel zum Verriegeln eine wiederverwendbare Verriegelungsanzeige aufweisen, die von dem Verbinder (6) oder dem Anschlussstück (7) getragen ist und die nach dem Trennen des Instrumentes (2) einen neuen Anschluss des Instrumentes an den Träger (3) gestattet.

15. Medizinisches Gerät nach einem der Ansprüche 1 bis 14, **dadurch gekennzeichnet, dass** die Betätigungsstange (11) ein flexibles Element wie ein Kabel oder ein starres Element wie eine starre Achse ist.

16. Medizinisches Gerät nach einem der Ansprüche 1 bis 15, **dadurch gekennzeichnet, dass** das abnehmbare medizinische Instrument das ganze oder einen Teil eines Operationswerkzeugs, eines Katheters, eines Endoskops oder einer Sonde bildet.

17. Medizinisches Gerät nach Anspruch 15, **dadurch gekennzeichnet, dass** das medizinische Instrument (2) ein Endoskop oder ein Katheter ist, das oder der eine röhrenförmige Umhüllung (G) aufweist, die an dem Verbinder (6) befestigt ist, und an deren Ende mindestens eine Betätigungsstange (11) befestigt ist, um die Ausrichtung des Kopfes der Umhüllung zu gewährleisten.

18. Medizinisches Gerät nach Anspruch 17, **dadurch gekennzeichnet, dass** die röhrenförmige Umhüllung (G) mindestens ein Organ (61) zum optischen, elektrischen Anschluss, zum Fluidanschluss oder zum Anschluss für einen mechanischen Durchgang zur Einweg-Verwendung aufweist und das komplementär zu mindestens einem Organ (60) zum optischen, elektrischen Anschluss, zum Fluidanschluss oder zum Anschluss für einen mechanischen Durchgang ist, das in dem Anschlussstück (7) montiert ist.

## Claims

1. A medical apparatus including a medical instrument **(2)** separable with respect to an actuating support **(3),** the device including a connecting and disconnecting system **(4)** between the instrument **(2)** and the support **(3),** including:
- a connector **(6)** supported by the instrument and including a guide **(9)** for at least one actuating rod **(11)** provided with a head **(12)** protruding at the free end of the guide, the connector **(6)** being provided with a rotational coupling member **(20),** and
- a connecting tip **(7)** supported by the support and including at least one engagement mechanism **(29),** this engagement mechanism (29) being equipped with a rotational coupling member **(33)** complementary with respect to the rotational coupling member **(20)** of the connector **(6),** the tip **(7)** and the connector **(6)** occupying a connection position after they are brought axially closer together leading to the rotational coupling between the connector **(6)** and the engagement mechanism **(29), characterized in that**:
- the head **(12)** of the actuating rod **(11)** has a semi-hemispherical or prismatic shape, kept elastically bearing on a transverse end face **(9₁)** of the guide, by means of a return spring **(17),**
- the tip **(7)** having at least one hook **(36)** connected to an actuating system **(37)** and having a housing **(38)** for receiving the head **(12)** of the rod to provide a translational connection between the rod **(11)** and the actuating hook **(36),**
- the engagement mechanism **(29)** is a cam guided in rotation and defining a recess **(30)** for receiving the head **(12)** of the actuating rod **(11),** the cam being mounted to extend in the position awaiting connection, inside the hook **(36)** with the recess **(30)** situated outside the hooks so as, following the axial approach movement, to receive the head **(12)** of a rod **(11)** released relative to the guide **(9),** the axial approach being followed by a rotational movement between the connector **(6)** and the connecting tip **(7)** to drive the rotation of the engagement mechanism **(29),** which moves the head **(12)** of the rod until it cooperates with the hook **(36)** order to provide a mechanical connection of the head **(12)** of the rod with the actuating hook **(36),** the engagement mechanism **(29)** extending, after rotation, outside the hook **(36),** which is then positioned inside the recess **(30)** so as to be able to slide freely.

2. The medical apparatus according to claim 1, **characterized in that** at least one hook **(36)** has a prismatic housing able to receive a complementary prismatic rod head **(12)** to ensure a translational and/or rotational connection between the rod **(11)** and the actuating hook **(36).**

3. The medical apparatus according to claim 2, **characterized in that** the hook **(36)** has a positioning slot **(41)** for the rod, arranged between the receiving housing **(38)** of the head and the free end of the hook.

4. The medical apparatus according to one of claims 1 to 3, **characterized in that** the connecting tip **(7)** is provided with at least one optical, electrical, fluid, or mechanical passage connecting member **(60),** and **in that** the guide **(9)** of the connector is provided with at least one complementary connecting member **(61)** that is respectively optical, electrical, fluid, or for mechanical passage, suitable for being aligned with the connecting member **(60)** of the tip, following the mechanical connection between the rod **(11)** and the actuating hook **(36).**

5. The medical apparatus according to claim 4, **characterized in that** the connecting member **(60)** crosses through the engagement mechanism **(29)** by means of a recess **(30)** adapted so that said member **(60)** is not moved by the engagement mechanism during its rotation, said member **(60)** being established substantially at the free end of the hook.

6. The medical apparatus according to one of claims 1 to 5, **characterized in that** the support **(3)** includes a body **(52)** cut out to have a series of small bars **(53),** at least one of which is linearly movable relative to the other small bars, so as to constitute a guideway that is part of the actuating system **(37)** of a hook **(36).**

7. The medical apparatus according to claim 6, **characterized in that** each small bar **(53)** forming a guideway is provided with a hook **(36)** at one of its ends while its other end is connected to a translational actuating member.

8. The medical apparatus according to claim 6, **characterized in that** at least one small bar **(53)** incorporates a rotational and/or translational actuating system.

9. The medical apparatus according to claim 6, **characterized in that** at least one small bar **(53)** provides for the positioning of an optical, electrical, fluid, or mechanical passage connecting member.

10. The medical apparatus according to one of claims 1 to 9, **characterized in that** the connector **(6)** and the connecting tip **(7)** include angular indexing means allowing their alignment in a predetermined position for their axial approach.

11. The medical apparatus according to claim 10, **characterized in that** the connector **(6)** and the tip **(7)** include means **(42, 43, 44)** for guiding slidingly for the axial approach, then rotationally to a stop position corresponding to the connection position of the connecting and disconnecting system **(4).**

12. The medical apparatus according to one of claims 1 to 11, **characterized in that** the connector **(6)** and the tip **(7)** include locking means for locking the connector and the tip in their connecting position.

13. The medical apparatus according to claim 11, **characterized in that** the locking means **(42, 43, 44)** include a single-use locking indicator supported by the connector, and which, after disconnection of the instrument following its use, prohibits a new connection of the instrument to the support.

14. The medical apparatus according to claim 12, **characterized in that** the locking means include a reusable locking indicator supported by the connector **(6)** or by the connecting tip **(7)**, and which, after disconnection of the instrument **(2),** allows a new connection of the instrument to the support **(3).**

15. The medical apparatus according to one of claims 1 to 14, **characterized in that** the actuating rod **(11)** is a flexible element such as a cable, or a rigid element such as a rigid shaft.

16. The medical apparatus according to one of claims 1 to 15, **characterized in that** the separable medical instrument constitutes all or part of a surgical tool, catheter, endoscope or lead.

17. The medical apparatus according to claim 15, **characterized in that** the medical instrument **(2)** is an endoscope or catheter including a tubular sheath **(G)** fixed on the connector **(6)** and at the end of which at least one actuating rod **(11)** is fixed to ensure the orientation of the head of the sheath.

18. The medical apparatus according to claim 17, **characterized in that** the tubular sheath **(G)** includes at least one optical, electrical, fluid, or mechanical passage connecting member **(61)**, for a single use and complementary to at least one respective optical, electrical, fluid or mechanical passage connecting member **(60),** mounted in the connecting tip **(7).**
